# EUROPEAN PATENT SPECIFICATION

(11) **EP 3 308 170 B1**
(45) Date of publication and mention of the grant of the patent: **23.08.2023**
(21) Application number: 16812316.4
(22) Date of filing: 15.06.2016
(51) Int. Cl.: C12M 1/00, C12M 3/00, C12M 1/12

(54) **ASEPTIC SAMPLE TRANSPORT POD**
ASEPTISCHER PROBENTRANSPORTBEHÄLTER
CAPSULE ASEPTIQUE DE TRANSPORT D'ÉCHANTILLONS

(30) Priority: 15.06.2015 US 201562175823 P
(43) Date of publication of application: 18.04.2018
(73) Proprietor: Entegris, Inc., Billerica, MA 01821 (US); Tokyo Electron Limited, Tokyo 107-6325 (JP); Sinfonia Technology Co., Ltd., Tokyo 105-8564 (JP)
(72) Inventor: BORES, Gregory, Prior Lake, MN 55372 (US); GOMI, Hisashi, Tokyo 107-6325 (JP); OZAKI, Shigenori, Nirasaki city Yamanashi 407-0192 (JP); TAMURA, Akitake, Tokyo 107-6325 (JP); FUJI, Toshimitsu, Tokyo 107-6325 (JP); TAKEUCHI, Haruki, Tokyo 405-8564 (JP)
(74) Representative: Greaves Brewster LLP
(86) International application number: PCT/US2016/037588
(87) International publication number: WO 2016/205341

(56) References cited:
- WO-A1-2012/156682
- WO-A1-2013/045659
- JP-A- 2007 097 481
- JP-A- 2011 160 840
- JP-A- 2013 204 910
- US-A- 4 111 753
- US-A1- 2003 044 991
- US-A1- 2007 281 351
- US-A1- 2009 029 450
- US-A1- 2011 107 788

## Description

### TECHNICAL FIELD

The field of the disclosure relates to containment, storage, transport, and automated transfer of biological materials such as cell cultures in trays in a safe, consistent, and controlled manner, particularly in automated biological material centers such as cell cultivation factories. The invention is defined in the claims.

### BACKGROUND

Conventional transfer equipment for transferring biological matter, such as culture trays, in an aseptic container to another aseptic environment is conventionally manually accomplished in an ad hoc manner, piece by piece. See for example Patent documents US4111753, US2011107788, JP2011160840 or JP2013204910. In particular, US4111753 discloses a transport unit in which the door includes a gasket to ensure that an airtight seal is formed between the door and the frame and also includes a chamber door latch operable from the inside of the chamber of the incubation apparatus, to hold the door in a closed position. Automation and robotic means for accomplishing such transfers in high volumes or in batches would be welcome.

### SUMMARY

The instant disclosure relates to storing and transporting culture vessels in an aseptic containment and robotic and/or automated transferring of batches of vessels of biological material, to other aseptic or controlled environments in a laboratory system such as a biological material handling facility, for cultivating, processing, growing, storing, examining, and/or analysis of biological material, all while maintaining aseptic integrity.

The sample transport pod (30) of the present invention comprises a container portion (40) having a pod door frame (92) defining downwardly facing pod door opening (94), and a pod door (42) that fits within and closes the downwardly facing pod door opening (94) for providing an aseptic interior, the pod door having an upper surface for receiving a cassette (50) for biological vessels, the pod door having a latch mechanism (104) with a central wheel (106) partially rotatable about a central axis and a plurality of radially extending latching linkages (105) connecting to the central wheel, the latching linkages movably radially outward and radially inward as the central wheel is partially rotated in one direction for engagement and disengagement of the latching linkages with the pod door frame, wherein the central wheel is a cam wheel with a plurality of cam surfaces (257.2) for engagement of the latching linkages, each latching linkage having a distal latching member (108) for engagement and disengagement with the pod door frame.

The sample transport pod (30) is in combination with a laboratory work station (187) having a load port (32), wherein the central wheel (106) of the sample transport pod (30) has a robotic handle (251) with structure that cooperates with a motorized robotic actuator (252) in the load port (32) for rotating the central wheel (106) of the latch mechanism (104).

When used herein, "vessels" includes conventional culture trays, with or without covers, and other containers or holders of cells. When used herein "laboratory system" may include a discrete work station, or a plurality of work stations, utilized for any of or for any combination of handling, cultivating, processing, growing, storing, examining, and/or analysis of biological material including cell cultures. In embodiments, the disclosures relate to a manually and robotically transferrable sample transport pod for biological material including a cassette therein configured to retain stacks of culture vessels such as trays.

The sample transport pod has a container portion with a door opening and a door that cooperates with and sealingly latches to the container portion at the door opening. The door opening faces downward and the door has an upwardly facing surface defining a receiving region with structure which receives a rack or cassette of vessels for holding biological materials, for example, culture trays. The receiving region may support the cassette in the z direction by gravity and constrain the cassette in the x and y directions by the structure of the receiving region of the door. The cassette may be secured to the door with mechanical latches, catches, cooperating structure, or magnetic couplings. The trays may be retained in the cassette such as by gravity and simple constraints in the horizontal (x-y) plane for securing one or more vertical stacks or collections of culture vessels stored therein. The interior of the pod is treatable to be aseptic such as, for example, with hydrogen peroxide gas, gamma sterilization, or other sterilization means known to those skilled in the art.

The pod may have manual handles and a central upper robotic handling flange on the container portion for facilitating handling and transport,

In embodiments, the sample transport pod is in combination with a laboratory work station (187) having a load port (32), wherein the central wheel of the sample transport pod has a robotic handle (251) with structure that cooperates with a motorized robotic actuator (252) in the load port for rotating the central wheel of the latch mechanism.

In embodiments, the pod interfaces with a load port on laboratory work station having equipment such as incubators, or stockers, or analysis stations. The pod has a lower base portion, the lower base portion defining a door frame that has a door opening and a door received in the door opening. The door seals and latches to the lower base portion. In embodiments, a door frame flange has a generally circular outer periphery and provides a generally circular door opening with the outer periphery of the door being generally circular. The door may have a latch mechanism with three distal latching members that extend outwardly from or at the door periphery to engage the door frame thereby latching the door to the door frame, and defining an interior chamber. The lower base portion of the pod may be secured to the load port such as by mechanical means and/or magnetic means.

In an embodiment, the base portion of the pod is secured to the load port. The base portion of the pod may be secured at multiple locations equally spaced around the flange. In one example, the base portion of the pod is secured to the load port at three locations. The pod base may include a plurality, such as three, inwardly extending peripheral recesses of the pod base that may cooperate with structure on the load port receiving collar. The pod base seats on the floor of the load port in a circular recess. Means, such as latch mechanisms, magnetic couplings, cooperating structure where the pod base is rotated with respect to the load port to secure the two together, may be provided for securing the pod in the z direction.

The load port includes central port door that is registered with the pod door and that has a door latch operating mechanism that engages the door latch mechanism of the door. The central port door also may have pod door retention means such as magnet means or other mechanical means for securing the pod door to the load port door and sealing features to seal the non aseptic upper surface of the port door to the non aseptic lower surface of the pod door, thereby sealingly containing or substantially containing the non aseptic surfaces between the respective doors.

With the pod secured at the load port, with the pod door secured to the port door, and with the cassette seated on the pod door, automated/robotic mechanisms may then unlatch the pod door from the base portion and lower the pod door and port door as a unit away from the container portion and into an operational area of the laboratory equipment. In the operational area, the biological material in the vessels in the cassette is then available for further operations, transfers, and/or storage. The non aseptic surfaces, although now located in the operational area, remain contained between the respective doors.

Purge lines, associated with the load port, can provide conditioned air, either intermittent or continuous, to the interior of the pod when the pod is situated on the load port. Separate purge stations, without operating port doors, can also be utilized to provide such conditioned air or gases. Such conditioned air, such as temperature controlled air or gases can have regulated amounts of, for example, O₂, H₂O, and/or CO₂, and can provide temperature control, filtering and/or removal of potential biological contaminants and other contaminants, such as VOC's. Stockers can similarly retain quantities of the pods, loaded and unloaded in controlled environments with purging of conditioned air. The pods can include valving and port(s) to allow charging or pressurization of the interior chamber. In an embodiment, the container portion may have a charging port that interfaces with a gas line. The charging port and cooperating gas line can each have a check valve and filter. When used herein, "check valve" may be a pressure actuated or a member actuated valve. For example, the gas line may have a valve member that upon engagement with the purge port opens the valve. The charging or purge inlet ports may have a pressure actuated valve that opens upon the pressurization of the port by the gas line.

In some examples, the biologic pod may include sensors therein for monitoring environmental conditions, for example, levels of O₂, H₂O, and/or CO₂, temperature and other environmental parameters. The sensor(s) may communicate externally electronically to a central controller, or network, or may display data relating to the conditions on or at the pod.

In some examples, temperature may be controlled inside the pod using temperature controlled purge air or gases and/or having a dual-wall pod with thermal insulation material between the inner and outer walls of the pod, such as silica aerogel or polyurethane. As such, temperatures may be maintained during transport or temporary storage. Other temperature control means such as self-contained environmental units may be attached to or contained within the container portions.

In some examples, the pods have a volume of 4 liters to 100 liters. In embodiments the pods have a volume of 1 liters to 15 liters. In embodiments the pods have a volume of 1 liters to 6 liters. In embodiments, the pods have an internal volume of .5 liters to .5 cubic meters.

In some examples, the biologic pods may be formed of injection molded or vacuum formed polymers. Suitable polymers may include, for example, polycarbonates, polystyrenes, polyethylenes, polyethylene terephthalate (e.g., PET, PETE, or PETG), acrylonitrile butadiene styrene (ABS), as well as metals such as aluminum and injection molded metals. Polymer blends may also be used. The polymers may include biocide additives for control of undesired biological agents. Additionally or alternatively, the polymers may have additives for imparting certain characteristics such as, for example, conductivity. Multiple polymers may be assembled or combined such as by overmolding. The pods may be opaque for blocking UV (ultra violet) light and/or visible light, and any wavelengths that can degrade or destroy biological matter contained within the cell culture vessels. In other embodiments, the pods may be transparent and still capable of clocking certain wavelength rays of light depending on the desired application. In such configurations, a transparent window for viewing the interior of the pod may be provided.

### BRIEF DESCRIPTION OF THE DRAWINGS

FIG. 1 is a perspective view of a bottom opening pod with loaded cassettes interfacing with a load port on a piece of equipment in accordance with embodiments of the invention.
FIG. 2 is a perspective view of a bottom opening pod, with the door separated and a cassette loaded with vessels in accordance with embodiments of the invention.
FIG. 3 is a cross-sectional view through a bottom opening pod revealing cassettes and a latch mechanism in accordance with embodiments of the invention.
FIG. 4 is a bottom perspective view of a bottom opening pod in accordance with embodiments of the invention.
FIG. 4A is a cross-sectional detail of a sample transport pod with the pod door separated from the pod door frame.
FIG. 4B is a cross sectional detail of the sample transport pod of FIG. 4A with the pod door seated in the door frame
FIG. 5 is a schematic view of a laboratory/cell processing facility that incorporates and utilizes embodiments of the inventions herein.
FIGS. 6 - 8 are schematic views of a station with a load port and a bottom loading pod integrating therewith in accordance with embodiments of the invention.
FIGS. 9 - 14 are schematic views of an overhead transport in conjunction with a bottom loading pod and station with a load port in accordance with embodiments of the invention.
FIG. 15 is a partially exploded view of a bottom opening pod with a base separated from the shell portion, the door in the door frame, and a cover plate removed from the door revealing the cam wheel of the latch mechanism in accordance with embodiments of the invention.
FIG. 16 is a detail view of the pod door with the latch cover removed revealing the door latch mechanism in accordance with embodiments of the invention.
FIG. 17 is a plan view of a latch arm and latch member of the latch mechanism in accordance with embodiments of the invention.
FIG. 18 is a cross-sectional view taken at line 18-18 of FIG. 17.
FIG. 19 is a cross-sectional view similar to FIG. 18 but with the latch arm fully engaged with the latch surface of the door frame causing the downward rotation of the latch arm in accordance with embodiments of the invention.
FIG. 20 is a perspective view of a load port that interfaces with the pod of FIGS. 2-6 in accordance with embodiments of the invention.
FIG. 21 is a cross-sectional view of the load port of FIG. 20 with the load port door seated in the load port door frame.
FIG. 22 is a perspective view of a bottom opening pod with transparent window locations illustrated in an otherwise opaque container portion in accordance with embodiments of the invention.
FIG. 23 is a cross-sectional and schematic view of the container portion charging port and a cooperating gas line.
FIG. 24 is a schematic plan view of a pod door on a load port door in a piece of equipment illustrating a purging, cleaning, or sterilizing method in accordance with embodiments of the invention.

### DETAILED DESCRIPTION OF EMBODIMENTS

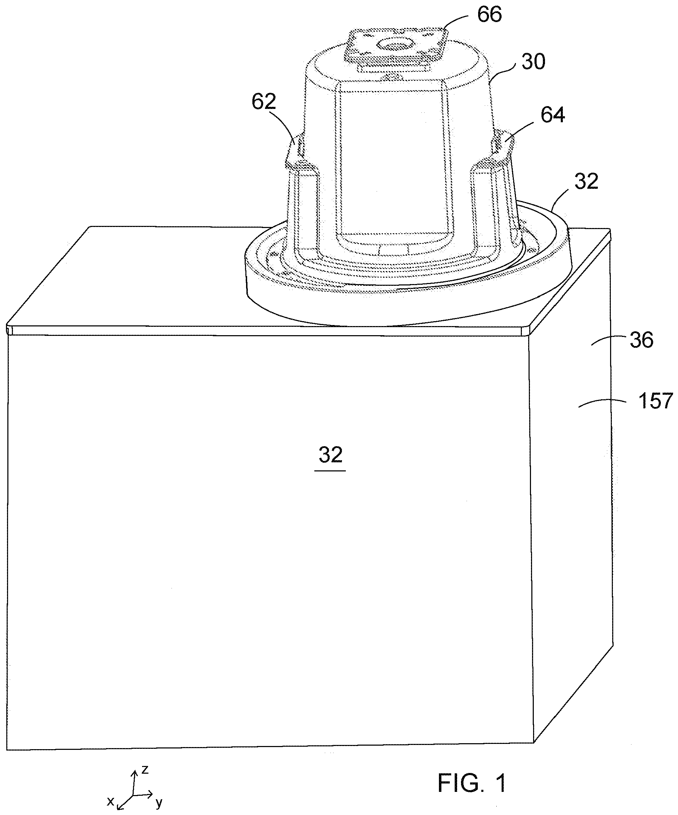

Referring to FIG. 1, a bottom opening pod 30 with an aseptic interior is depicted on a load port 32, as part of a biological work station 34. The work station has a containment structure 36 defining an aseptic interior and may be one of several pieces of equipment used in cell processing laboratories, such as cell culture incubators or feeders for providing nutrition to the cell cultures.

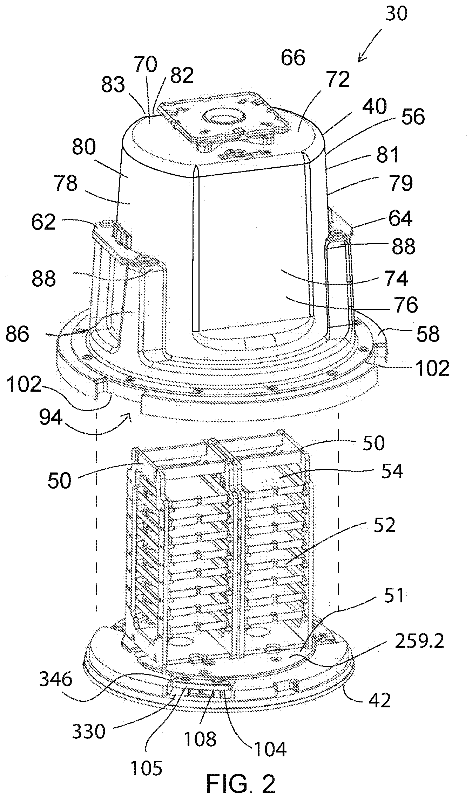

Referring to FIGS. 2-4B, details of the bottom opening pod 30 are illustrated. The pod 30 generally includes a container portion 40, and a pod door 42, that cooperate to define an open interior 43 which contains one or more culture vessel cassettes 50 seated on an upwardly facing surface 51 of the door. In some cases, a pair of cassettes are seated on the door. Spaced stacks of culture vessels 52 are constrained within slots 53 in the vessels. The vessels 52 may have biological material 54 such a cell cultures or growth media therein. The container portion has a shell portion 56 extending from a base or pod door frame 58. In embodiments, a pair of manual handles 62, 64, and a robotic flange 66 are part of the container portion. In embodiments, the shell portion may be separately formed from the base and the components assembled together using fasteners 67. Similarly, the robotic flange 66 and handles 62, 64 may be attached with fasteners to the shell portion. In other embodiments, the shell portion 56 and base 58 may be unitarily formed with the handles and robotic flange attached thereto. In embodiments the shell portion 56, base 58, handles 62, 64 and robotic flange 66 may all be formed integrally such as by injection molding utilizing a single mold with the shell portion, base, handles, and robotic flange all included in a single mold.

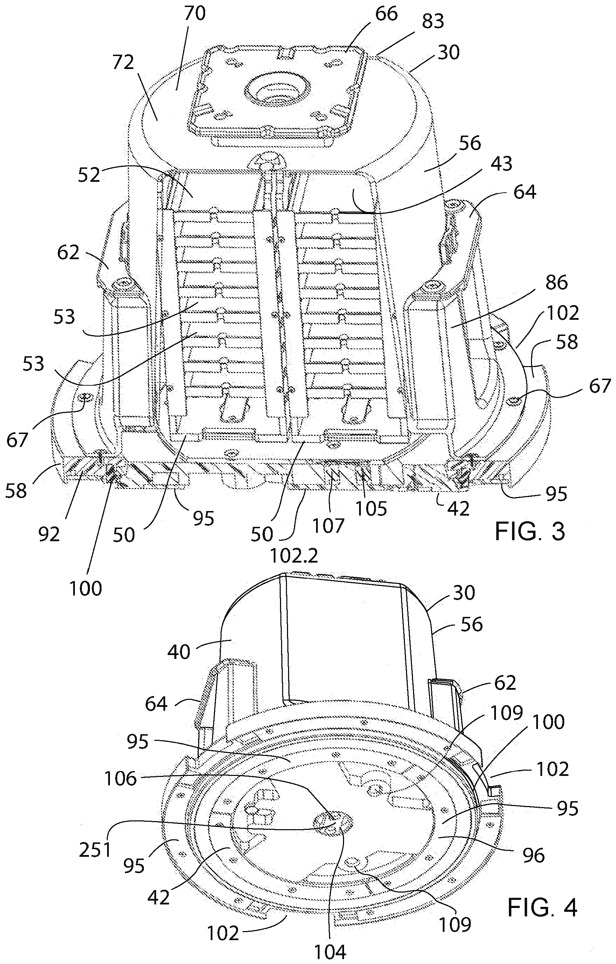

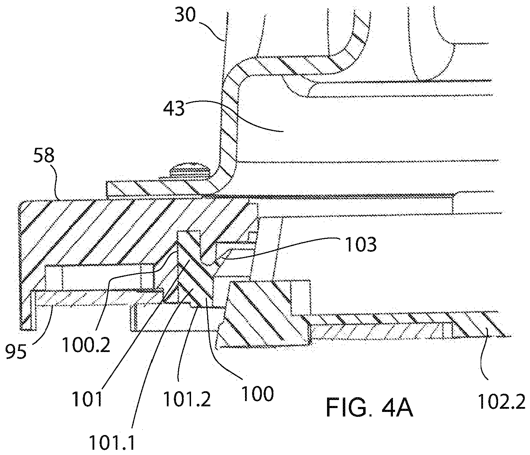

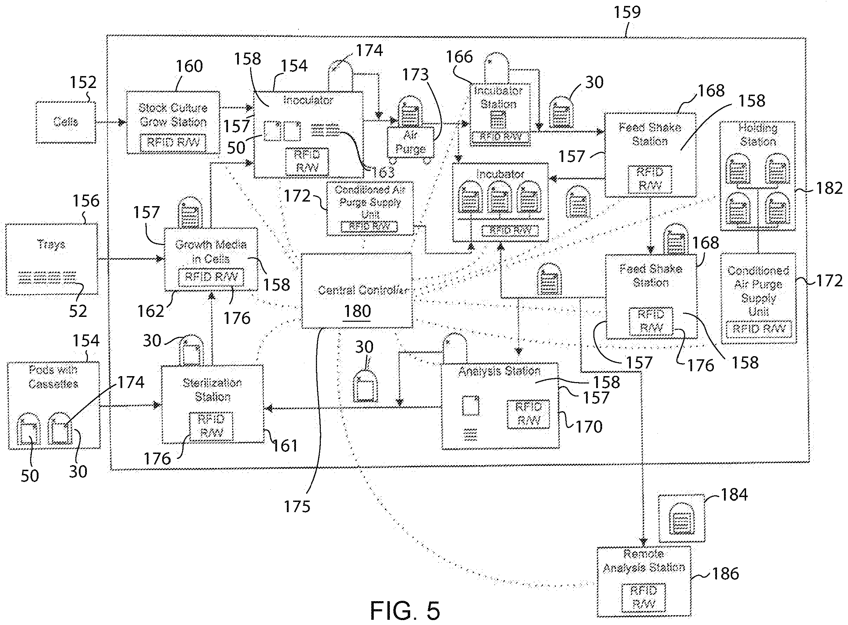

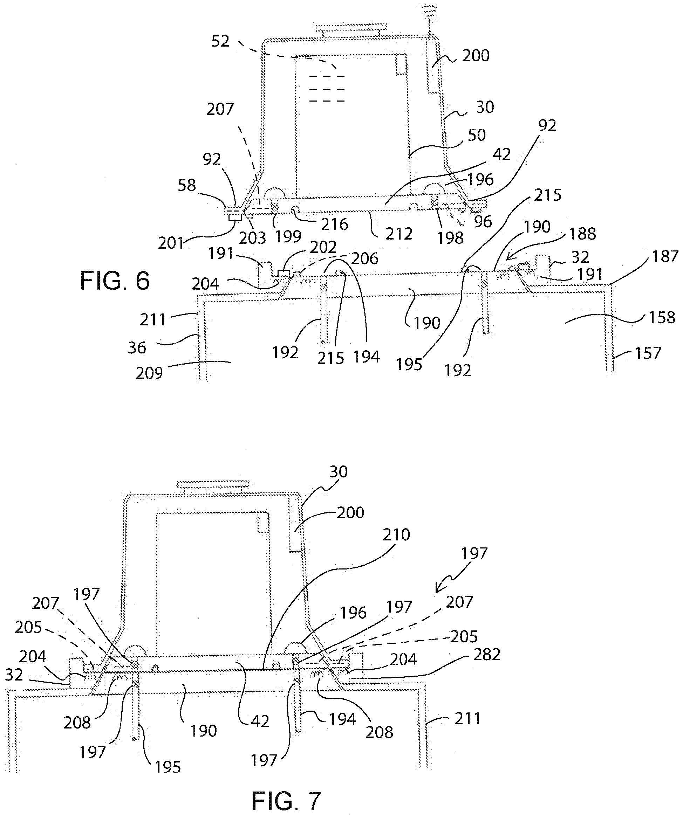

Referring to FIGS. 1-4B and 6-7, the container portion 40 generally has a closed top 70 with a top wall 72, a closed forward side 74, a flat forward wall 76, closed sides 78, 79, arcuate side walls 80, 81, closed back side 82, and a flat back wall 83. Outwardly protruding structures 86 provide attachment structure with shoulders 88 for attaching the manual handles 62, 64. The base 58 of the container portion 40 provides the door frame 92 and door opening 94 that faces downwardly as illustrated in FIG. 2. A circular seal 100 having a V-shaped or Y-shaped cross section is secured to the pod door frame 58 and seals with the pod door 42 when the pod door is seated in the pod door frame. As shown in FIG. 4A, the seal 100 has a post portion 101 and a cantilevered leg 103 both extending from a seal base portion 101.1 In one embodiment, the post portion 101 forms one leg of the V-shaped seal and engages in a downwardly facing groove 100.2 of the base 58. The cantilevered leg 103 forms the other leg of the V-shaped seal. Note that post portion 101 of the seal has a downwardly facing surface 101.2 which may be utilized as a sealing surface between the pod door frame 58 and the load port door frame 191 shown in FIG. 6. Other seals may also be appropriate. The pod door 42 can have circular portions of magnetically attractable material 95 such as steel plates or other ferrous containing material. The circular portions define an armature 96 for attraction to the electromagnets in the load port door 190 thereby providing a controllable selective magnetic coupling. Such coupling can be controlled by a central controller 180 as shown in FIG. 5 Proximity of the circular armature 96 to the circular seals facilities security and tightness of the pod door. See discussion referencing FIGS. 6-8 below.

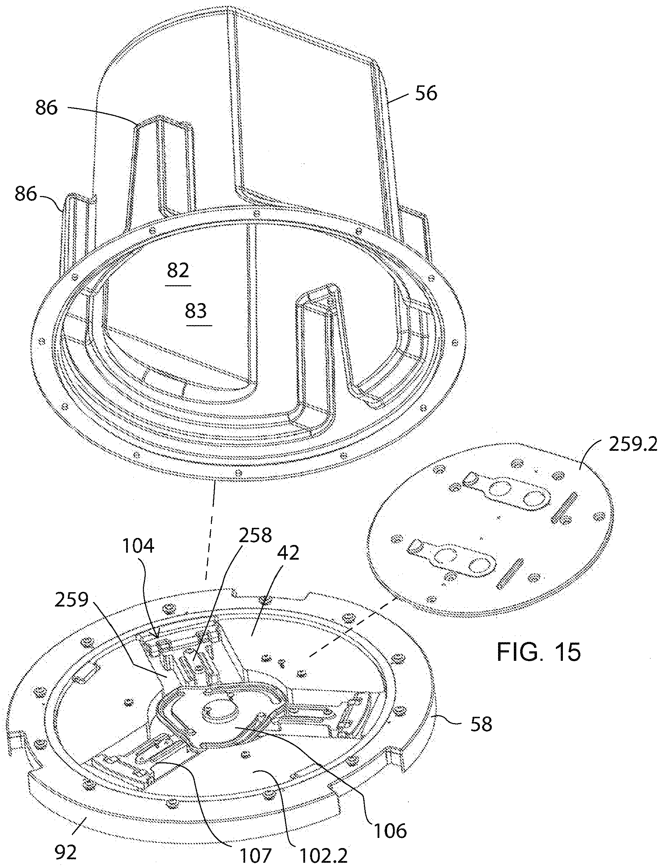

Continuing to refer to Figures 2-4B, and referring to FIG. 15, the door 42 includes a chassis 102.2 supporting a latch mechanism 104 with a partially rotatable central wheel 106, configured as a cam wheel, and three latching linkages 105 that include three latch arms 107 with pivotally attached latching members 108 that extend from the periphery of the pod door 42 to latch onto, engage, the pod door frame 92 securing the pod door 42 therein, and to disengage, releasing the pod door 42 from the pod door frame 92.

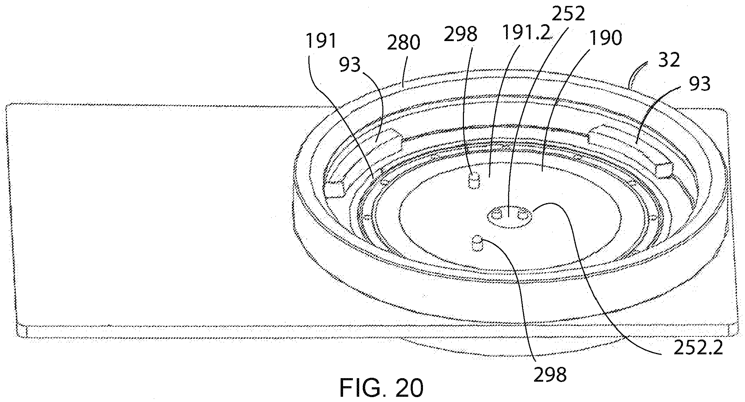

The pod door 42 may have a structure, such as pin recesses or openings 109 that cooperate with structure on the load port door 190 for proper alignment between said load port door 190 and pod door 42. The door frame 92 may have outer peripheral notches or openings 102 that correspond to projections 93 on the load port, see FIG. 20, for facilitating proper alignment of the bottom opening pod 30 with the load port 32.

Referring to FIG. 5, a schematic drawing of an exemplary cell culture facility 159 for cell culture growing and processing is illustrated and includes a number of laboratory systems and laboratory work stations that have containment structures 157 with interiors 158 that receive the doors with cassettes of the bottom opening pods 30, and that provide operations to the culture vessels. The operational interiors are intended to remain aseptic. Shipments coming into the facility, shown on the left side of the figure, include the cells 152 to be cultured and upon which experiments will be performed, a supply 154 of the bottom opening pods with cassettes, and a supply 156 of vessels 52 for holding the cell cultures and that are to be carried in the cassettes. The stock cells, as received, are grown and multiplied at the grow station 160.

Upon receipt at the cell culture facility, bottom opening pods 30, are typically sterilized at the sterilization station 161. In embodiments, the surfaces in the interior of the pod are intended to be sterile and aseptic. This includes the inside surface of the shell portion and the surfaces of the door that face upwardly or are exposed to the interior of the pod. Such sterilization may be by hydrogen peroxide gas or by conventional autoclaving or other known means.

Growth media may be added to pre-sterilized culture vessels 52 at station 162. The culture vessels may be loaded into cassettes 50 as batches 163 at this station and then transferred to the inoculator station 164. At station 164, the stock cells are inoculated into the growth media in the culture vessels 52 under intended aseptic conditions in the interior of the inoculator station with the vessels removed from the cassettes. The batches are replaced into the cassettes, the cassettes returned to the bottom opening pods, and the door of the bottom opening pod latched onto the respective container portions. From the inoculator station 164, the cell cultures may be transferred to an incubator station 166 by way of the closed pod where the cell cultures in the vessels are allowed to grow. The vessels 52, by way of the bottom opening pods, may be periodically transferred to feed and shake stations 168 where the vessels will be removed from the cassettes and liquid food provided to the cultures. The batches, depending on the experiment protocols, may loop within the incubating and feeding stations for several steps. At some point, the vessels 52 and batches may be transferred by way of the pods to an analysis station 170, all the while maintaining the intended aseptic conditions, where the cultures may be analyzed consistent with the pending experiments. Upon completion of the experiment, the bottom opening pods may be returned to the sterilization station before further use.

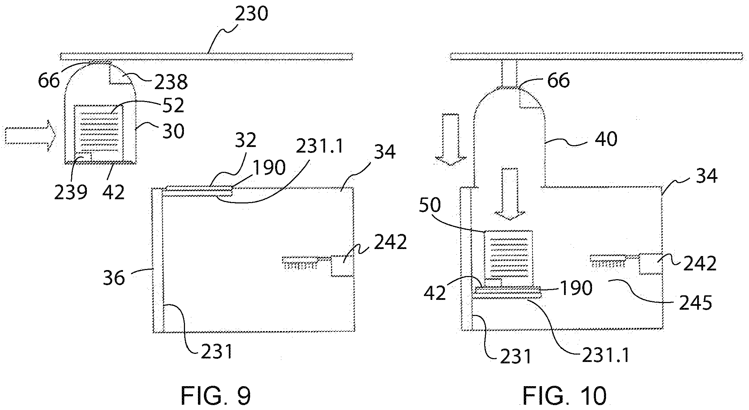

Although it is known to control environments within incubators and other laboratory work stations, the embodiments herein include controlling the environment within the pods and also for the vessels 52 and batches in the pods. In this sense, the individual pods may become effective micro incubators. The conditioned air purge supply unit 172 can provide conditioned air, where O₂, humidity levels, and/or CO₂ levels, and other gas levels, and temperature are controlled. The air may be distributed to the individual stations and/or to the loaded pods. In embodiments, a transportable air purge unit 173 may be used to transport pods with batches therein and provide interior environmental control. As discussed below, sensors monitoring the gas and moisture levels may be located in the pods. The bottom opening pods and cassettes may each be equipped with RFID tags 174, indicated by the asterisks on the pods in FIG 5. Alternately, labels with bar codes and other code that is machine readable code such as QR codes, may be utilized. Each station or other locations, may be equipped with RFID/bar code or QR code readers and/or writers 176 for tracking the pods and/or batches 163 of vessels, and/or individual vessels 52. The readers and/or writers communicate with the central controller 180 which may be an appropriately programed general purpose computer. Said central controller 180 can also automate the processes by controlling the individual stations, the conditioned air, and the transfer of pods, discussed below with reference to FIGS. 9-14, as well as monitoring conditions, sample/pod/cassette locations and statuses throughout and acting thereupon. A tracking system 175 can include the central controller 180 and the RFID tags 174 or other identifiers to track the pods 30. Storage stockers or holding stations 182 may store the pods with or without cell culture batches. The holding stations may also be provided with conditioned air purge from an air supply unit 172.

In certain instances, a pod with a batch or batches of cell cultures and in a box 184 may be shipped to a remote analysis station 186.

Referring to FIGS 6-8, the methodology of transferring the cassettes into and out of the bottom opening pods 30 is illustrated along with environmental control features of the pods and work stations. A non-specific laboratory work station 187 has the containment structure 36 that defines the interior 158. The load port 32 is on top of the containment structure thereby providing access to the interior 158 of the work station 187. In FIG. 6, the pod 30 with the cassette 50 loaded with vessels 52 is being transferred to the load port 32. The pod is seated in the receiving region 188 with the pod door directly above and within the footprint of the load port door 190, and with the base 58 and door frame 92 of the pod directly above the load port door frame 191. The load port may include purge lines 192 that may include an inlet purge port 194 and an outlet purge port 195. In embodiments, controllable valves 197, either operated by the central controller 180 or by pressure as check valves, may be located in the pod door and in the load port door 190. A diffuser 196 connected to a purge pod inlet 198 may eject the conditioned air into the pod and a counterpart connected to a purge pod outlet 199 exhausts the purge from the pod. The purge lines 192 can extend to a conditioned air purge supply unit 172 separate from the work station or an air purge conditioning unit 199.2 that is part of the work station. The conditioning unit 199.2 may also provide conditioned air to the interior 158 of the work station 187 with an outlet 199.4 to the interior and a return air inlet 199.6. Supplemental lines 199.7 can provide air, gas, water, electricity, control lines to the conditioning unit and work station. Sensor(s) 200.2 can provide monitoring of the interior of the workstation and by cable or wirelessly transmit data regarding the sensed conditions to the central controller 180 and/or to a dedicated controller 199.9 at the workstation.

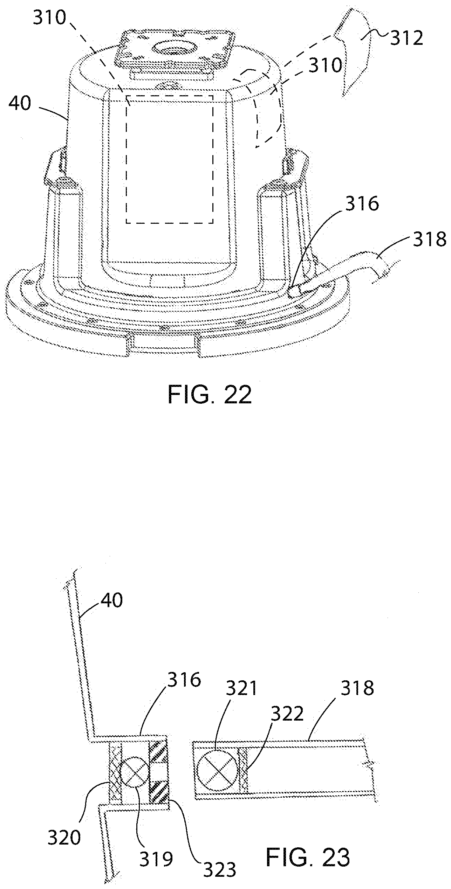

In embodiments, the pod can include one or more sensor(s) 200, located in the interior of the pods, or at least exposed to the interior of the pods, that are adapted to monitor environmental conditions in the pod and can communicate to the central controller 180 by cable or wirelessly for monitoring and recording environmental conditions and controlling operation of the conditioned air units as needed or appropriate to modify those conditions. Specific environmental conditions sensed and controlled can include but not limited to: temperature, humidity, gas levels, microorganism levels, light levels. The inlet purge port 194 of the purge port may deliver a variety of gases for environmental control and can also deliver gases for sterilization. In embodiments, the purge inlet 198 and purge outlet 199 on the pod may be on the container portion with check valves exteriorly exposed. See also FIGS. 22 and 23.

Continuing to refer to FIGS. 6-8, the pod may seal to the load port 32 with sealing structure 201 on the pod base 58 and cooperating sealing structure 202 on the load port door frame 191 which will isolate the aseptic interior 209 of the work station 187 from the exterior. Such sealing structures may comprise an elastomeric seal and a seal engagement surface or more rigid sealing surfaces. Similarly, cooperating sealing structure 203, 206 may be on the pod door and load port door 190 to isolate the non-aseptic surfaces 210 of the pod door 42 and load port door 190 from the aseptic interior 209.

In FIG. 7, electromagnets 204, may cooperate with metal or ferrous materials 205 in or on the base of the container portion 42 for securing the pod to the load port 32. The electromagnets may be controlled by the central controller 180 or may be controlled by the load port at the load port. Similarly, electromagnets 208 in the load port door 190 may be controlled by the central controller 180 to cooperate with metal or ferrous material 207, the armature 96, on or in the pod door to couple the pod door to the load port door 190 when the pod is seated thereon, or such can be controlled at the load port.

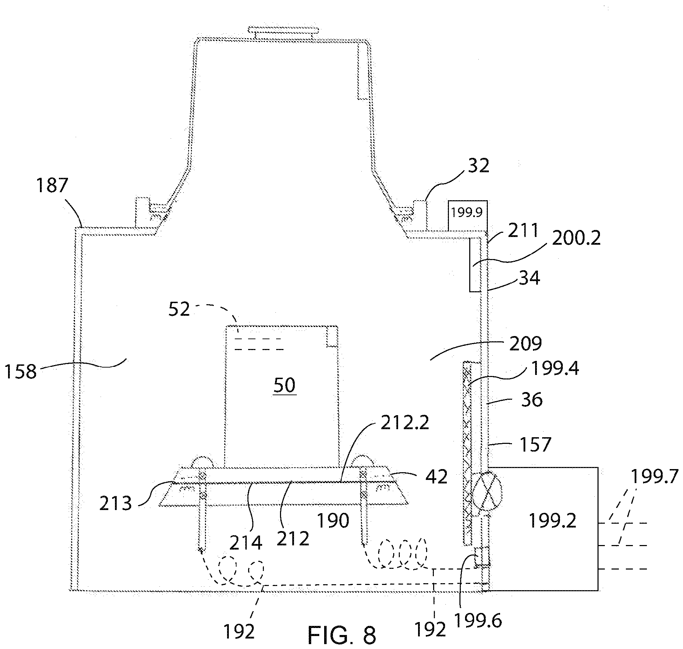

Referring to FIG. 8, the load port door 190 with the coupled pod door 42 and with the cassette seated on the pod door, is lowered into the aseptic interior 158 of the station 211 for accessing the culture vessels 52 in the cassette 50. The non-aseptic condition on the exterior 212 of the pod door 42 and the exterior 212.2 of the load port door 190, are secured and contained between the two doors. Seals 213, may be utilized to provide desired integrity to the sealing off of the contained non aseptic region 214. The seals may be cooperating engagement surfaces and may include elastomeric or flexible circular seals.

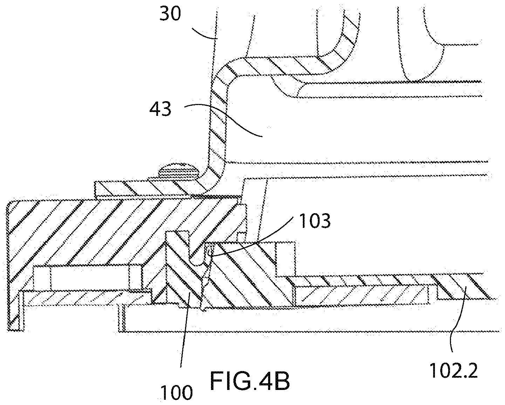

Proximity of the metal or ferrous material, such as the armature 96 in FIG. 4, to the circular seal structure between the load port door 190 and pod door 42 facilitates security and tightness of the pod door 42 to the load port door 42.

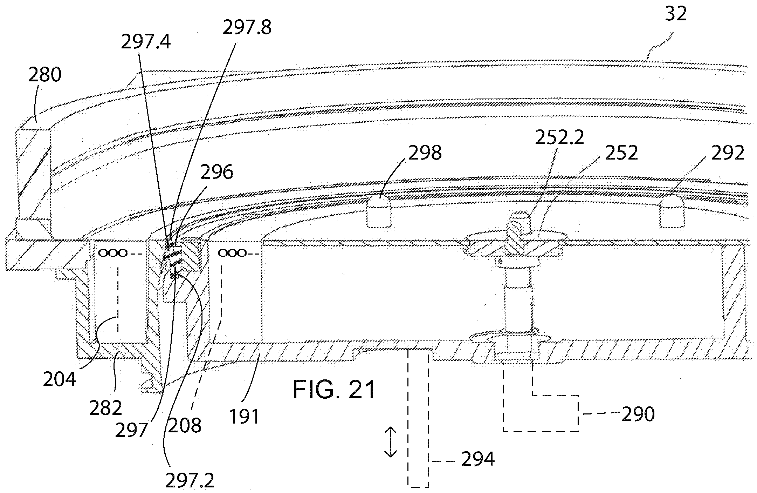

Continuing to refer to FIGS. 6-8, in embodiments, cooperating alignment structures 215, 216 may facilitate proper alignment of the pod door 42 with the load port door 190 when the pod 30 is seated in the load port 32. Such alignment structure can be provided by corresponding pins and recesses, as illustrated, or other suitable projections and recesses/openings. See, for example, the pin recess 109 in FIG. 4 and the pins 298 illustrated in FIGS. 20 and 21.

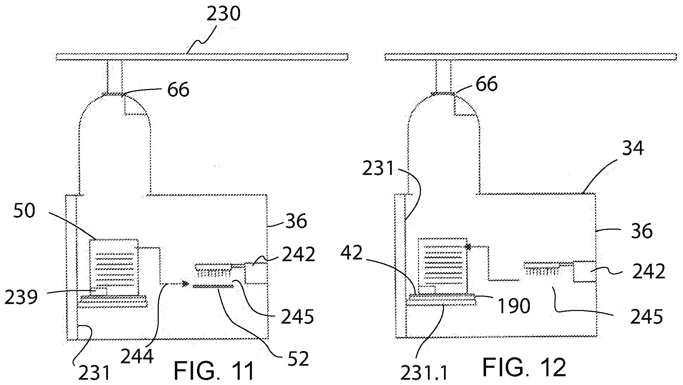

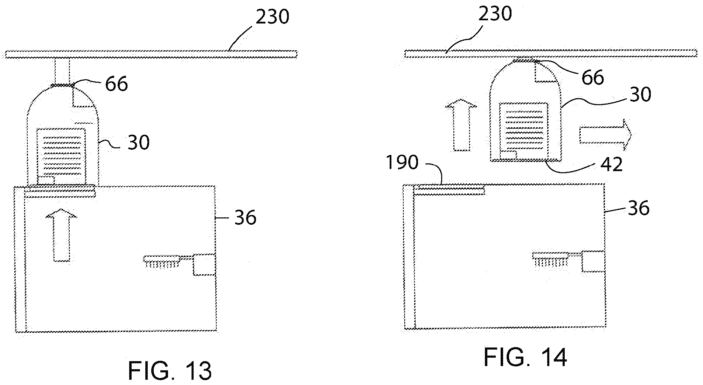

Referring to FIGS. 9-14, a simplified series of drawings illustrate transfer operations of the bottom loading pods 30. The pods have upper robotic flanges 66 for grasping by conveyance system 230. The conveyance system 230 is controlled by the central controller 180 and can deliver such pods 30 by way of elevated tracks, to load ports 32 at particular work stations 34 and at such work stations 34 the central controller 180 may lower the respective pod to the load port 32, and then lower the load port door 190 and pod door 42 by way of the elevator 231 including the elevator platform 231.1, as shown by FIG. 10. RFID tags or other position indicator sensor means 238, 239 can provide information to the central controller 180 as to the location of the respective pods and/or cassettes. FIG. 11 illustrates a cell culture vessel 52 being removed from a cassette for performing an operation thereon by an automated component 242, for example, providing nutrients. Internal robotic transfer mechanisms 244 grasp a vessel 52 and move it to the operation region 245 and return it to the cassette as illustrated in FIG. 12. The cassette 50 is returned to the pod 30 by way of the pod door being reinserted into the container portion. FIGS. 13 and 14 illustrate the pod door and load port door 190 being uncoupled and the conveyance system conveying the pod to the next location.

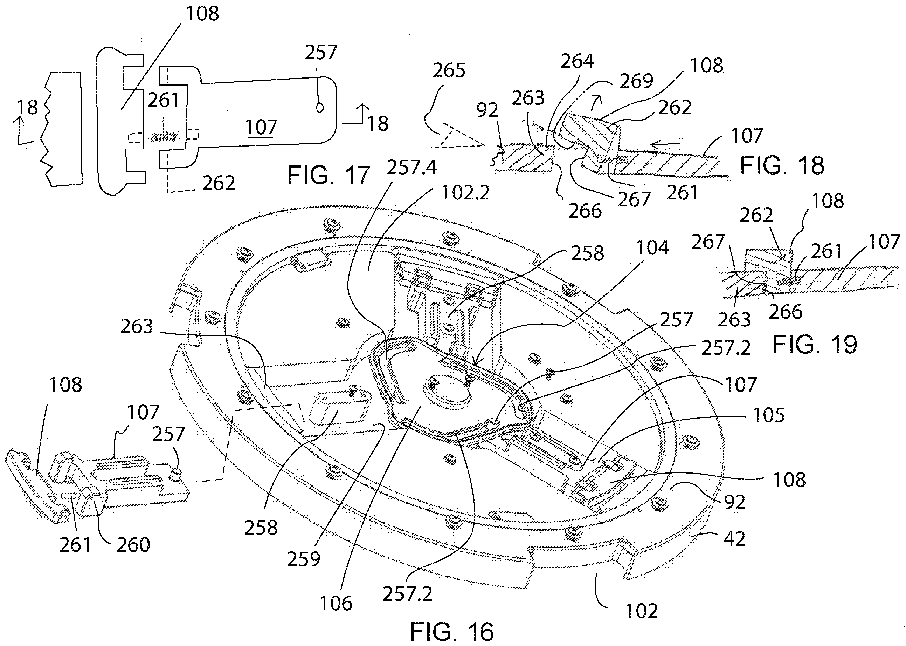

Referring to FIGS. 3-4B and 15-20, further details of the pod door latch mechanism 104 are illustrated. The central wheel 106 is rotatable by way of a robotic handle 251,that faces downward and interfaces with a robotic actuator 252 having pair of pins 252.2 on the load port, see FIG. 20 and discussion below. Configurations and cooperating structure other than a pair of pins and pair of cooperating apertures may be utilized such as a single male member and aperture, the male member and aperture cooperating non cylindrical shapes. The cammed wheel 106 engages three latch arms 107 at cam followers 257 to move the latch arms 107 radially inward and outward as the cam followers 257 follow the cam surfaces 257.2 in the cam openings 257.4. In some cases, the latch arms 104 are equally spaced from each other and around the circular pod door 42. In some cases the latch mechanism 104 may include a spring 261. The latch arms 107 are each constrained in the horizontal, x-y plane, by a respective central guide member 258. The latch mechanism 104 is constrained in a latch mechanism cavity 259 in the pod door chassis 102.2 and covered by a latch cover 259.2 In some cases, the latch arms 107 pivotally connect to a latching member 108 through a pivot point 260 that defines a horizontal axis 262 about which the latching member 108 rotates. The spring 261 may provide a force to rotate the latching member 108 upwardly. The rotation is constrained to an acute angle 265 such that the upward rotation is quite limited. The rotation does, however, allow the latching member 108 to present an inclined surface 269 inclined upwardly from horizontal for the initial engagement with the cooperating latching ledge 263 and surface 264 of the pod door frame 92. See FIG. 18. This provides some tolerance in the latch components coming together. Once the latching members 108 fully engages the latching ledges and surfaces by way of full outward radial extension of the latch arm 107, the abutting surfaces 266, 267, see FIG. 19, of the latching member 108 and latch arm 107 cause the latching member 108 to rotate downwardly to "square up" and to provide a downward force on the latching surface 264 of the door frame upon full engagement with the pod door frame 92.

Referring to FIGS. 4, 20, and 21, the load port 32 has a receiving region 191.2 within the collar 280. The load port includes a load port door 190 in a load port door frame 191. Robotic actuator 252 is configured to interface with the robotic handle 251 of the cammed wheel 106 of the pod door latch mechanism 104 for rotating the cammed wheel 106 and latching or unlatching the pod door 42. The robotic actuator is rotated by way of a motor 290 (shown , schematically) controlled by the central controller 180. The load port door 190 is lowered by way of robotic mechanisms 294 (shown schematically). A seal 296 engaged with the port door keeps the region below the load port isolated from the exterior environment. Seal 296 can be a V-shaped seal with one leg of the V-shaped seal defining a post portion 297 secured in a downwardly extending groove 297.2. The two legs of the V-shaped seal extending from a base portion 297.4. An upwardly facing surface 297.8 may provide the seal between load port door 190 and the pod door when the pod is seated on the load port 32. Upwardly projecting structures configured as pins 298 cooperate with pin recesses 109 on the robotic handle 251 at the bottom of the pod door, such as shown in FIG. 4, to position and secure the door facilitating operation of the latch mechanism.

When the pod is seated on the load port, the peripheral recesses or notches 102, see FIG. 4, may be aligned with collar structure whereby the pod may seat and be constrained in the receiving region. Electromagnets, controlled by the central controller 180 attract ferrous metal on the bottom opening pod to the load port. The outer electromagnet cooperate with metal on the base of the container portion to secure the container portion on the load port while the inner electromagnet attracts metal on the pod door and can effectively couple the pod door to the load port door 190.

Referring to FIG. 22, the dashed lines illustrate a transparent window 310 in an otherwise opaque shell portion for minimizing or eliminating UV exposure of the batches. The windows may be formed by first molding the window portion then inserting the window in the mold for the container portion shell portion and overmolding an opaque material on the window. The window can be covered by an opaque cover 312 when the contents are not being viewed. When not in use, the cover can pivot up or engage the container portion proximate the margins of the window. Features can be provided on the shell portion for removably fastening the cover thereto.

Referring to FIG. 22 and 23, the container portion can also have air vents or "charging" ports 316 for pressurizing or purging the interior of the pod with suitable gas combinations by way of a gas line 318 in flow communication with the interior of the pod. A check valve 319 and filter 320 can be situated in the charging port 316. Additionally the gas line may have a check valve 321 and filter 322. A suitable seal 323, such as an elastomeric member, shown on the charging port, may also or alternatively be on the gas line. It is also contemplated that the container portion may have environmental control module attached thereto and accessing the interior of the pod and working with sensors in the pod to maintain suitable conditions.

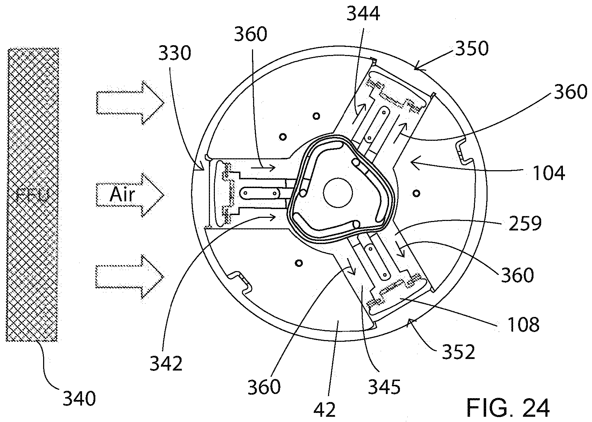

Referring to FIG. 2 and 24, the work stations may have a forced gas supply such as internal forced filtration units 340 which provide filtered air or other gases that may be conditioned. The pod door 42 when retracted downwardly into the station may be oriented as shown to allow forced air (or other gases) flow from the forced filtration unit to enter the latch mechanism 104. Direct confrontational alignment of the direction of the forced air(or other gases) with one of the latch openings 330 allows the forced air(or other gases) to enter one latch arm cavity 342 and then flow through to the other latch arm cavities 344, 345 defining a gas flow pathway 360 and then exit the other two latch openings 350, 352. See the arrows representing the air flow direction. The forced air can be sterilizing gases such as hydrogen peroxide gas or steam. In embodiments, other access for the forced air or gases may be provided in the door other than the latch openings. Also, the latch openings may include upwardly facing openings 346 whereby the forced air or other gases may circulate above the pod door, for example into a cassette 50 positioned thereon.

## Claims

1. A sample transport pod (30) comprising a container portion (40) having a pod door frame (92) defining a downwardly facing pod door opening (94), and a pod door (42) that fits within and closes the downwardly facing pod door opening for providing an aseptic interior, the pod door having an upper surface for receiving a cassette (50) for biologic vessels,
the pod door having a latch mechanism (104) with a central wheel (106) partially rotatable about a central axis and a plurality of radially extending latching linkages (105) connecting to the central wheel, the latching linkages movable radially outward and radially inward as the central wheel is partially rotated in one direction and then the opposite direction for engagement and disengagement of the latching linkages with the pod door frame,
wherein the central wheel is a cam wheel with a plurality of cam surfaces (257.2) for engagement of the latching linkages, each latching linkage having a distal latching member (108) for engagement and disengagement with the pod door frame.

2. The sample transport pod of claim 1, wherein each latching linkage comprises the distal latching member and a latch arm (107) engaged with the cam wheel and to which the distal latching member is pivotally connected about a horizontal axis, the latching member biased to rotate upwardly an acute angle thereby presenting an inclined surface (269) for an initial engagement with the pod door frame, the latching member rotatable downwardly upon full engagement with the pod door frame.

3. The sample transport pod of claim 1, in combination with a laboratory work station (187) having a load port (32), wherein the central wheel of the sample transport pod has a robotic handle (251) with structure that cooperates with a motorized robotic actuator (252) in the load port for rotating the central wheel of the latch mechanism.

4. The sample transport pod of any of claim 1-3, in combination with a load port (32), wherein the central wheel of the sample transport pod has a robotic handle (251) with structure that cooperates with a motorized robotic actuator (252) for rotating the central wheel.

5. The sample transport pod of claim 1, wherein the downwardly facing pod door opening is generally circular and has an axis, and wherein the pod door is generally circular, and wherein the pod door engages the pod door frame in an axial direction, and wherein the pod door confronts the pod door frame at a converging inwardly facing annular surface that narrows in an upwardly direction, and wherein the pod door frame has a flexible circular seal (100) attached thereto, the seal having, in cross-section, an upwardly and axially extending post portion (101) that seats in a downwardly facing groove (100.2), and a cantilevered finger (103) extending inwardly and upwardly from a lower portion of the post portion, wherein when the pod door is not in the door frame, the finger extends inwardly away from the pod door frame, and when the pod door is seated in the pod door frame, the pod door chassis engages the seal at the lower post portion and at the cantilevered finger, and wherein the cantilevered finger is deflected outwardly.

6. The sample transport pod of claim 1 wherein a circular seal (100) secured to the pod door frame provides a sealing engagement between the container portion and the pod door when the pod door is seated in the pod door frame, the circular seal having a downwardly facing exposed surface positioned for sealing with a load port door frame.

7. The sample transport pod of claim 1 wherein the container portion comprises a shell portion (56) formed of a polymer composition that is opaque and blocks ultraviolet light.

8. The sample transport pod of claim 1, wherein the latching members (108) of the latching linkages extend out latch openings (330, 350, 352) for engaging the door frame thereby securing the pod door in the door frame, wherein when the pod door is separated from the container portion, one of the latch openings (330) providing a gas entry into the pod door with another of the latch openings (350, 352) providing a gas exit out of the pod door, the pod door configured to provide a gas pathway (360) between the gas entry and the gas exit.

9. The sample transport pod of claim 8 in combination with a laboratory work station (187) having a load port (32), the pod seatable on the load port, the load port having a load port door (190) that latches to the pod door and unlatches the pod door from the pod door frame, the load port door and pod door lowerable into an interior of the laboratory work station, the laboratory work station further comprising a forced gas supply (340) that directs forced gas horizontally toward the lowered load port door and pod door for providing the gas entry into the pod door.

## Patentansprüche

1. Probentransportbehälter (30), umfassend einen Behälterabschnitt (40) mit einem Behältertürrahmen (92), der eine nach unten gerichtete Behältertüröffnung (94) begrenzt, und einer Behältertür (42, die in die nach unten gerichtete Behältertüröffnung hineinpasst und diese verschließt, um einen aseptischen Innenraum bereitzustellen, wobei die Behältertür eine Oberseite zur Aufnahme einer Kassette (50) für biologische Gefäße aufweist,
wobei die Behältertür einen Verschlussmechanismus (104) mit einem zentralen Rad (106) aufweist, das teilweise um eine zentrale Achse drehbar ist und eine Vielzahl sich radial erstreckender Verschlussstangen (105) aufweist, die mit dem zentralen Rad verbunden sind, wobei die Verschlussstangen radial nach außen und radial nach innen beweglich sind, wenn das zentrale Rad teilweise in eine Richtung und dann in die entgegengesetzte Richtung zum Eingreifen und Lösen der Verschlussstangen mit dem Behältertürrahmen gedreht wird,
wobei das zentrale Rad ein Nockenrad mit einer Vielzahl von Nockenflächen (257.2) zum Eingriff der Verschlussstangen ist, wobei jede Verschlussstange ein distales Verschlusselement (108) zum Eingreifen und Lösen mit dem Behältertürrahmen aufweist.

2. Probentransportbehälter nach Anspruch 1, wobei jede Verschlussstange das distale Verschlusselement und einen Verschlussarm (107) umfasst, der mit dem Nockenrad in Eingriff steht und mit dem das distale Verschlusselement um eine horizontale Achse schwenkbar verbunden ist, wobei das Verschlusselement so vorgespannt ist, dass es sich in einem spitzen Winkel nach oben dreht, wodurch eine geneigte Fläche (269) für einen anfänglichen Eingriff mit dem Behältertürrahmen angeboten wird, wobei das Verschlusselement bei vollständigem Eingriff mit dem Behältertürrahmen nach unten drehbar ist.

3. Probentransportbehälter nach Anspruch 1, in Kombination mit einer Laborarbeitsstation (187) mit einer Ladeöffnung (32), wobei das zentrale Rad des Probentransportbehälters einen Robotergriff (251) mit einer Struktur aufweist, die mit einem motorisierten Roboteraktuator (252) in der Ladeöffnung zusammenwirkt, um das zentrale Rad des Verschlussmechanismus zu drehen.

4. Probentransportbehälter nach einem der Ansprüche 1 bis 3, in Kombination mit einer Ladeöffnung (32), wobei das zentrale Rad des Probentransportbehälters einen Robotergriff (251) mit einer Struktur aufweist, die mit einem motorisierten Roboteraktuator (252) zum Drehen des zentralen Rads zusammenwirkt.

5. Probentransportbehälter nach Anspruch 1, wobei die nach unten gerichtete Behältertüröffnung im Allgemeinen kreisförmig ist und eine Achse aufweist, und wobei die Behältertür im Allgemeinen kreisförmig ist und wobei die Behältertür in einer axialen Richtung mit dem Behältertürrahmen in Eingriff steht, und wobei die Behältertür dem Behältertürrahmen an einer konvergierenden, nach innen weisenden Ringfläche gegenübersteht, die sich nach oben hin verengt, und wobei am Behältertürrahmen eine flexible kreisförmige Dichtung (100) angebracht ist, wobei die Dichtung im Querschnitt einen sich nach oben und axial erstreckenden Pfostenabschnitt (101) aufweist, der in einer nach unten weisenden Nut (100.2) sitzt, und einen auskragenden Finger (103), der sich von einem unteren Abschnitt des Pfostenabschnitts nach innen und oben erstreckt, wobei sich der Finger nach innen vom Behältertürrahmen weg erstreckt, wenn sich die Behältertür nicht im Türrahmen befindet, und, wenn die Behältertür im Behältertürrahmen sitzt, das Behältertür-Chassis an der Dichtung am unteren Pfostenabschnitt und am auskragenden Finger angreift, und wobei der auskragende Finger nach außen gebogen ist.

6. Probentransportbehälter nach Anspruch 1, wobei eine am Behältertürrahmen befestigte kreisförmige Dichtung (100) für einen dichtenden Eingriff zwischen dem Behälterabschnitt und der Behältertür sorgt, wenn die Behältertür im Behältertürrahmen sitzt, wobei die kreisförmige Dichtung eine nach unten gerichtete freiliegende Oberfläche aufweist, die zur Abdichtung mit einem Ladeöffnungstürrahmen positioniert ist.

7. Probentransportbehälter nach Anspruch 1, wobei der Behälterabschnitt einen Schalenteil (56) umfasst, der aus einer Polymerzusammensetzung gebildet ist, die undurchsichtig ist und ultraviolettes Licht blockiert.

8. Probentransportbehälter nach Anspruch 1, wobei die Verschlusselemente (108) der Verschlussstangen sich aus Verschlussöffnungen (330, 350, 352) heraus erstrecken, um mit dem Türrahmen in Eingriff zu treten, wodurch die Behältertür im Türrahmen gesichert wird, wobei, wenn die Behältertür vom Behälterabschnitt getrennt ist, eine der Verschlussöffnungen (330) einen Gaseintritt in die Behältertür ermöglicht, während eine andere der Verschlussöffnungen (350, 352) einen Gasaustritt aus der Behältertür heraus ermöglicht, wobei die Behältertür so konfiguriert ist, dass sie einen Gasweg (360) zwischen dem Gaseintritt und dem Gasaustritt bereitstellt.

9. Probentransportbehälter nach Anspruch 8, in Kombination mit einer Laborarbeitsstation (187) mit einer Ladeöffnung (32), wobei der Behälter auf die Ladeöffnung aufsetzbar ist, und wobei die Ladeöffnung eine Ladeöffnungstür (190) aufweist, die an der Behältertür einklinkt und die Behältertür aus dem Behältertürrahmen ausklinkt, wobei die Ladeöffnungstür und die Behältertür in einen Innenraum der Laborarbeitsstation absenkbar sind, wobei die Laborarbeitsstation außerdem eine Gasdruckversorgung (340) umfasst, die Druckgas horizontal in Richtung auf die abgesenkten Ladeöffnungstür und Behältertür leitet, um für den Gaseintritt in die Behältertür zu sorgen.

## Revendications

1. Capsule de transport d'échantillons (30) comprenant une partie conteneur (40) possédant un cadre de porte de capsule (92) définissant une ouverture de porte de capsule orientée vers le bas (94), et une porte de capsule (42) qui s'ajuste à l'intérieur et ferme l'ouverture de porte de capsule orientée vers le bas pour fournir un intérieur aseptique, la porte de capsule possédant une surface supérieure destinée à recevoir une cassette (50)
pour des récipient biologiques,
la porte de capsule possédant un mécanisme de verrou (104) avec une roue centrale (106) pouvant tourner partiellement autour d'un axe central et une pluralité de tringleries de verrouillage s'étendant radialement (105) raccordées
à la roue centrale, les tringleries de verrouillage étant mobiles radialement vers l'extérieur et radialement vers l'intérieur tandis que la roue centrale est partiellement tournée dans un sens et ensuite dans le sens opposé pour la mise en prise et la séparation des tringleries de verrouillage avec le cadre de porte de capsule,
ladite roue centrale étant une roue à came avec une pluralité de surfaces de came (257.2) pour une mise en prise des tringleries de verrouillage, chaque tringlerie de verrouillage possédant un élément de verrouillage distal (108) en vue de la mise en prise et la séparation avec le cadre de porte de capsule.

2. Capsule de transport d'échantillons de la revendication 1, chaque tringlerie de verrouillage comprenant l'élément de verrouillage distal et un bras de verrou (107) en prise avec la roue à came et auquel l'élément de verrouillage distal est raccordé de manière pivotante autour d'un axe horizontal, l'élément de verrouillage sollicité pour tourner vers le haut selon un angle aigu présentant ainsi une surface inclinée (269) pour une mise en prise initiale avec le cadre de porte de capsule, l'élément de verrouillage pouvant tourner vers le bas lors d'une mise en prise complète avec le cadre de porte de capsule.

3. Capsule de transport d'échantillon de la revendication 1, en combinaison avec un poste de travail de laboratoire (187) possédant un port de charge (32), ladite roue centrale de la capsule de transport d'échantillon possédant une poignée robotique (251) avec une structure qui coopère avec un actionneur robotique motorisé (252) dans le port de charge pour faire tourner la roue centrale du mécanisme de verrou.

4. Capsule de transport d'échantillon de l'une quelconque des revendications 1-3, en combinaison avec un port de charge (32), ladite roue centrale de la capsule de transport d'échantillon possédant une poignée robotique (251) avec une structure qui coopère avec un actionneur robotique motorisé (252) pour faire tourner la roue centrale.

5. Capsule de transport d'échantillons de la revendication 1, ladite ouverture de porte de capsule orientée vers le bas étant globalement circulaire et possédant un axe, et ladite porte de capsule étant globalement circulaire, et ladite porte de capsule venant en prise avec le cadre de porte de capsule dans une direction axiale, et ladite porte de capsule faisant face au cadre de porte de capsule au niveau d'une surface annulaire orientée vers l'intérieur convergente qui se rétrécit dans une direction vers le haut, et ledit cadre de porte de capsule possédant un joint d'étanchéité circulaire souple (100) attaché à celui-ci, le joint d'étanchéité possédant, en coupe transversale, une partie montant (101) s'étendant axialement et vers le haut qui est placée dans une rainure orientée vers le bas (100.2), et un doigt en porte-à-faux (103) s'étendant vers l'intérieur et vers le haut à partir d'une partie inférieure de la partie montant, lorsque la porte de capsule n'est pas dans le cadre de porte, ledit doigt s'étendant vers l'intérieur en s'éloignant du cadre de porte de capsule, et lorsque la porte de capsule est placée dans le cadre de porte de capsule, ledit châssis de porte de capsule venant en prise avec le joint d'étanchéité au niveau de la partie montant inférieure et du doigt en porte-à-faux, et ledit doigt en porte-à-faux étant dévié vers l'extérieur.

6. Capsule de transport d'échantillons de la revendication 1, un joint d'étanchéité circulaire (100) fixé au cadre de porte de capsule fournissant une mise en prise d'étanchéité entre la partie conteneur et la porte de capsule lorsque la porte de capsule est placée dans le cadre de porte de capsule, le joint d'étanchéité circulaire possédant une surface exposée orientée vers le bas positionnée pour assurer l'étanchéité avec un cadre de porte de port de charge.

7. Capsule de transport d'échantillons de la revendication 1, ladite partie conteneur comprenant une partie coque (56) formée d'une composition polymère qui est opaque et bloque la lumière ultraviolette.

8. Capsule de transport d'échantillons de la revendication 1, lesdits éléments de verrouillage (108) des tringleries de verrouillage s'étendant hors des ouvertures de verrou (330, 350, 352) pour venir en prise avec le cadre de porte, fixant ainsi la porte de capsule dans le cadre de porte, lorsque la porte de capsule est séparée de la partie conteneur, l'une des ouvertures de verrou (330) fournissant une entrée de gaz dans la porte de capsule avec une autre des ouvertures de verrou (350, 352) fournissant une sortie de gaz hors de la porte de capsule, la porte de la capsule étant conçue pour fournir un passage de gaz (360) entre l'entrée de gaz et la sortie de gaz.

9. Capsule de transport d'échantillons de la revendication 8 en combinaison avec un poste de travail de laboratoire (187) possédant un port de charge (32), la capsule pouvant être placée sur le port de charge, le port de charge possédant une porte (190) de port de charge qui verrouille la porte de capsule et déverrouille la porte de capsule du cadre de porte de capsule, la porte de port de charge et la porte de capsule pouvant être abaissées dans un intérieur du poste de travail de laboratoire, le poste de travail de laboratoire comprenant en outre une alimentation en gaz forcé (340) qui dirige le gaz forcé horizontalement vers la porte de port de charge abaissée et la porte de capsule pour fournir l'entrée de gaz dans la porte de capsule.
